# EUROPEAN PATENT SPECIFICATION

(11) **EP 4 031 204 B1**
(45) Date of publication and mention of the grant of the patent: **26.11.2025**
(21) Application number: 19769778.2
(22) Date of filing: 16.09.2019
(51) Int. Cl.: A61M 1/36, G16H 40/60

(54) **SEMI-AUTOMATIC SAFETY CHECKS IN HLMS**
HALBAUTOMATISCHE SICHERHEITSPRÜFUNGEN IN HLMS
CONTRÔLES DE SÉCURITÉ SEMI-AUTOMATIQUES POUR COEUR-POUMON ARTIFICIEL

(43) Date of publication of application: 27.07.2022
(73) Proprietor: LivaNova Deutschland GmbH, 80939 Munich (DE)
(72) Inventor: SCHUBERT, Friedemann, 80639 Munich (DE); PENKA, Ottmar, 81245 Munich (DE)
(74) Representative: Grünecker Patent- und Rechtsanwälte PartG mbB
(86) International application number: PCT/EP2019/074603
(87) International publication number: WO 2021/052553

(56) References cited:
- US-A1- 2005 063 860
- US-A1- 2017 102 846

## Description

### TECHNICAL FIELD

The present disclosure relates to a semi-automatic safety check process for medical equipment, in particular, a semi-automatic safety checks in heart lung machines (HLMs).

### BACKGROUND

Typically, in extracorporeal perfusion, the perfusion system needs to be checked by the user before each use. This is usually done by a checklist, either on paper or electronically. This checklist is usually managed outside the HLM itself, either on paper or using electronic documentation systems.

Risk analysis of the HLM requires the check of some HLM components before the HLM is being used. This includes checking one or more Level Sensors, Bubble Detectors and Pressure monitors. Often, risk management requires a confirmation of the checks having been performed in the medical equipment (the HLM) itself. On the other hand, usability engineering requires not placing unnecessary burden on the user when interacting with the User Interface of the device for performing the checks. As such, an implementation of an additional checklist in the HLM is considered additional burden as the user needs to input/record several check results twice (in different locations).

US 2005/063860 A1 discloses a disposable, integrated extracorporeal blood circuit employed during cardiopulmonary bypass surgery and performing gas exchange, heat transfer, and microemboli filtering functions in a way as to conserve volume, to reduce setup and change out times, to eliminate a venous blood reservoir, and to substantially reduce blood-air interface. Blood from the patient or prime solution is routed through an air removal device that is equipped with air sensors for detection of air. An active air removal controller removes detected air from blood in the air removal device. A disposable circuit support module is used to mount the components of the disposable, integrated extracorporeal blood circuit in close proximity and in a desirable spatial relationship to optimize priming and use of the disposable, integrated extracorporeal blood circuit. A reusable circuit holder supports the disposable circuit support module in relation to a prime solution source, the active air removal controller and other components.

US 2017/102846 A1 discloses a customizable and intuitive user interface for medical systems and devices, such as cardiopulmonary bypass systems, perfusion systems, extracorporeal circulation apparatuses, and heart-lung machines, which allows for ease of access to critical patient data to facilitate blood perfusion and to monitor and regulate various physiological parameters of a patient and an extracorporeal blood flow circuit during surgical procedures, such as cardiopulmonary bypass and extracorporeal membrane oxygenation (ECMO).

### SUMMARY

It is provided a heart lung machine, HLM, comprising:
a control area network, CAN;
a pump communicatively coupled to the CAN;
a plurality of sensors, wherein each of the plurality of sensors is communicatively coupled to the CAN; and
a control assembly communicatively coupled to the CAN, the control assembly comprising a control display device and a processing unit, the processing unit configured to facilitate a semi-automatic safety check by:
   providing an HLM system safety check user interface, UI, on a display device, the HLM system safety check UI comprising a representation of each of the plurality of sensors;
   activating a first sensor of the plurality of sensors;
   presenting, via the UI, an indication of the activation of the first sensor;
   determining an alarm state of the first sensor;
   presenting a representation of the alarm state of the first sensor on the control display device;
   presenting a safety check result of the first sensor via the Ul;
   saving, in a data management system, DMS, or a system log of the HLM, a safety check result corresponding to the first sensor;
   activating a second sensor of the plurality of sensors;
   presenting, via the UI, an indication of the activation of the second sensor;
   determining an alarm state of the second sensor;
   presenting a representation of the alarm state of the second sensor on the control display device;
   presenting, via the UI, a safety check result of the second sensor; and
   saving, in the DMS or the system log of the HLM, a safety check result corresponding to the second sensor;
   wherein the processing unit is further configured to facilitate the semi-automatic safety check by:
      accessing a first alarm message, via the CAN, wherein the first alarm message is generated by the first sensor and indicates an alarm state of the first sensor; and
      determining the alarm state of the first sensor based on the first alarm message;
wherein the pump is configured to:
   receive the first alarm message; and
   provide, in response to receiving the first alarm message, a reaction message to the processing unit, the reaction message indicating that the pump acknowledged the alarm state of the first sensor; and
   perform, in response to receiving the first alarm message, an alarm system reaction, wherein the alarm system reaction comprises ceasing pump operation if the pump is running before receiving the first alarm message;
wherein the processing unit is further configured to:
   determine that the alarm system reaction performed by the pump in response to receiving the first alarm message from the first sensor has been executed as intended, thereby identifying a successful safety check result corresponding to the first sensor;
   present, via the UI, the successful safety check result; and
   enable the HLM to perform a perfusion task.

Further, it is provided a method of operating a heart lung machine, HLM, the HLM comprising a control area network (CAN), a pump communicatively coupled to the CAN, a plurality of sensors, wherein each of the plurality of sensors is communicatively coupled to the CAN, and a control assembly communicatively coupled to the CAN, the control assembly comprising a control display device and a processing unit, the method comprising:
providing an HLM system safety check user interface, UI, on a display device, the HLM system safety check UI comprising a representation of each of the plurality of sensors;
activating a first sensor of the plurality of sensors;
presenting, via the UI, an indication of the activation of the first sensor;
determining an alarm state of the first sensor;
presenting a representation of the alarm state of the first sensor on the control display device;
presenting, via the UI, a result of the safety check of the first sensor;
saving, in a data management system, DMS, or a system log, of the HLM, the safety check result corresponding to the first sensor;
activating a second sensor of the plurality of sensors;
presenting, via the UI, an indication of the activation of the second sensor;
determining an alarm state of the second sensor;
presenting a representation of the alarm state of the second sensor on the control display device;
presenting, via the UI, a result of the safety check of the second sensor; and
saving, in a data management system, DMS, or a system log, of the HLM, the safety check result corresponding to the second sensor.

While multiple embodiments are disclosed, still other embodiments of the presently disclosed subject matter will become apparent to those skilled in the art from the following detailed description, which shows and describes illustrative embodiments of the disclosed subject matter. Accordingly, the drawings and detailed description are to be regarded as illustrative in nature and not restrictive.

### BRIEF DESCRIPTION OF THE DRAWINGS

FIG. 1A is a front perspective view of an illustrative heart lung machine (HLM), in accordance with embodiments of the subject matter disclosed herein.
FIG. 1B is a side perspective view of the illustrative HLM depicted in FIG. 1A, in accordance with embodiments of the subject matter disclosed herein.
FIG. 1C is a partial perspective view of the base of the trolley depicted in FIGS. 1A and 1B, in accordance with embodiments of the subject matter disclosed herein.
FIG. 2 is a block diagram depicting an illustrative operating environment of an HLM, in accordance with embodiments of the subject matter disclosed herein.
FIG. 3 is a schematic block diagram depicting a safety check process in an illustrative operating environment of an HLM, in accordance with embodiments of the subject matter disclosed herein.
FIG. 4 depicts an illustrative screen shot depicting an HLM system mapping user interface (UI), in accordance with embodiments of the subject matter disclosed herein.
FIG. 5 is a flow diagram depicting an illustrative method of operating a heart lung machine (HLM), in accordance with embodiments of the subject matter disclosed herein.

The terms "up," "upper," and "upward," and variations thereof, are used throughout this disclosure for the sole purpose of clarity of description and are only intended to refer to a relative direction (i.e. , a certain direction that is to be distinguished from another direction), and are not meant to be interpreted to mean an absolute direction. Similarly, the terms "down," "lower," and "downward," and variations thereof, are used throughout this disclosure for the sole purpose of clarity of description and are only intended to refer to a relative direction that is at least approximately opposite a direction referred to by one or more of the terms "up," "upper," and "upward," and variations thereof.

Although the term "block" may be used herein to connote different elements illustratively employed, the term should not be interpreted as implying any requirement of, or particular order among or between, various blocks disclosed herein. Similarly, although illustrative methods may be represented by one or more drawings (e.g., flow diagrams, communication flows, etc.), the drawings should not be interpreted as implying any requirement of, or particular order among or between, various steps disclosed herein. However, certain embodiments may require certain steps and/or certain orders between certain steps, as may be explicitly described herein and/or as may be understood from the nature of the steps themselves (e.g., the performance of some steps may depend on the outcome of a previous step). Additionally, a "set," "subset," or "group" of items (e.g., inputs, algorithms, data values, etc.) may include one or more items, and, similarly, a subset or subgroup of items may include one or more items. A "plurality" means more than one.

### DETAILED DESCRIPTION

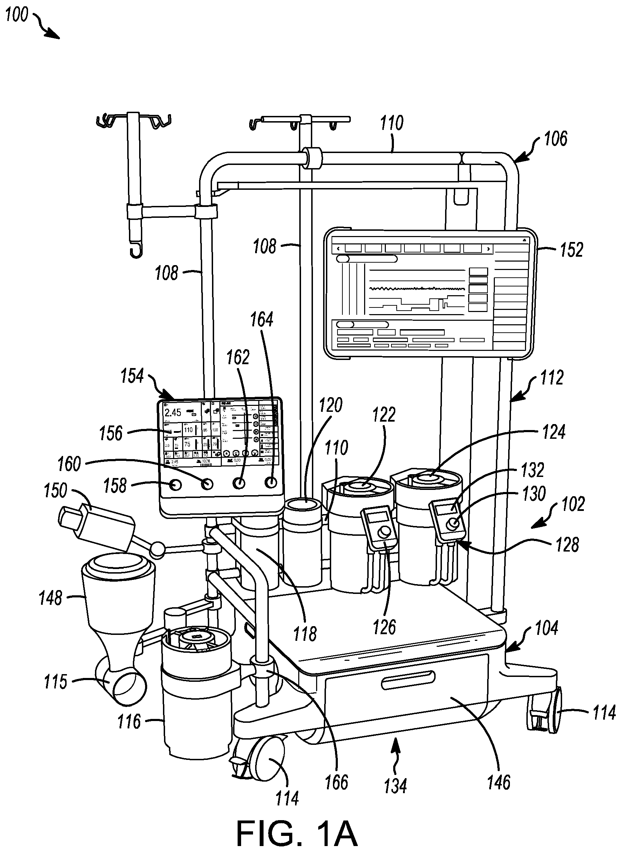

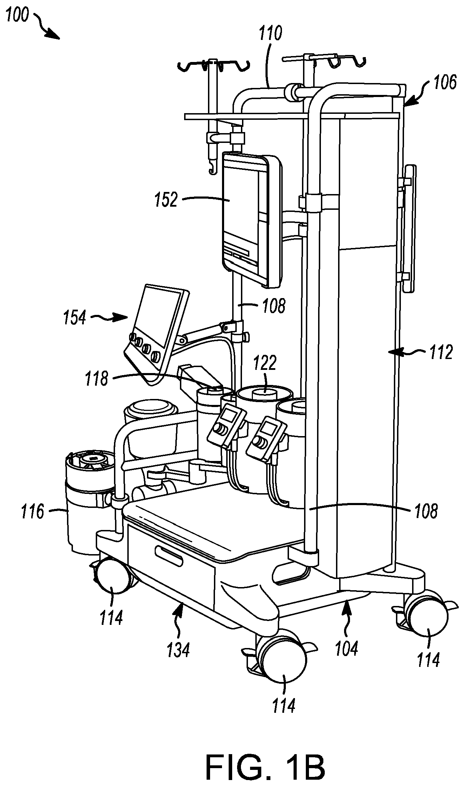

FIG. 1A is a front perspective view of an illustrative heart lung machine (HLM) 100, in accordance with embodiments of the subject matter disclosed herein; and FIG. 1B is a side perspective view of the illustrative HLM 100 depicted in FIG. 1A, in accordance with embodiments of the subject matter disclosed herein. As shown, the HLM 100 includes a trolley 102 having a base 104 that includes an internal cavity (not shown) for housing any number of different controls, electrical circuits, hydraulic circuits, a battery discharger, and/or the like. For example, in embodiments, a peripheral processing unit may be disposed within the base 104. A mast assembly 106 is coupled to the base 104 and extends upwards from the base 104. The mast assembly 106 may include any number of different mast components, including vertical poles 108, horizontal rails 110, and/or the like. In embodiments, the trolley 102 includes an enclosure 112 that is configured to facilitate cable management, provide A/C outlets, include a power switch for the HLM 100, include an extension box, and/or the like. As shown, the trolley 102 also may include wheels 114 coupled to the base 104.

As shown, the HLM 100 also may include a number of different types of components such as an oxygenator 115 (which may actually be considered to be an element of an extracorporeal circuit used with the HLM, but may be referred to herein as being a component of the HLM due to being connected to the trolley 102); pumps 116, 118, 120, 122, 124; and/or the like. In embodiments, one or more of the components 115, 116, 118, 120, 122, and 124 (and/or others) may be coupled to any number of different portions of the mast assembly 106, and may include, for example, an exposed actuator control unit (ACU). For example, as shown, pumps 122 and 124 may each include an exposed ACU 126 and 128, operably connected thereto, respectively. As shown, an ACU 128 may include a control knob 130 configured to receive user input (e.g., manipulation of the knob 130) for controlling operation of the pump 124, and an information display device 132 configured to present information associated with the pump such as, for example, one or more parameters (e.g., measured device parameters such as, for instance, flow, rpm, etc.). According to embodiments, an ACU may be configured to facilitate control of a pump, a motorized clamp, a motorized occluder, an infusion device, and/or any number of other types of devices that may be associated with an HLM.

Traditionally, HLMs have utilized roller pumps that are each integrated into a modular console component. The modular console components are stacked next to one another on the base of an HLM to provide an array of pumps. The modular console component also houses an ACU having an interface for controlling the corresponding integrated roller pump. One advantage of having the ACU interface provided at the modular console component is that, during an emergency situation, the perfusionist can easily determine the ACU that corresponds to a particular roller pump. More recently, mast mounted roller pumps (without the modular console component housing) have been utilized in HLMs. Mast mounted pumps provide more flexibility in the configuration of the HLM; however, if the ACUs for the mast mounted pumps are located remotely, or detached from, the mast mounted pumps, it's potentially more difficult for the perfusionist (i.e., the user) to identify the ACU that controls a particular pump.

Embodiments of the present disclosure include mast mounted roller pumps, such as pumps 122 and 124 of FIG. 1A, having corresponding ACUs, such as ACUs 126 and 128, respectively. The ACUs 126 and 128 are attached, connected, or otherwise operatively coupled to the corresponding mast mounted roller pumps 122 and 124. The connectedness, or close proximity of the ACU to the mast mounted roller pump allows the user to precisely determine the ACU that controls a particular mast mounted roller pump in a high pressure, or emergency situation, including a situation where the control display device 156 is not functioning properly or is disabled. Thus, the ability to mount the pumps 122 and 124 to the mast assembly 106 allows a much wider range of configurations to meet the user's particular needs.

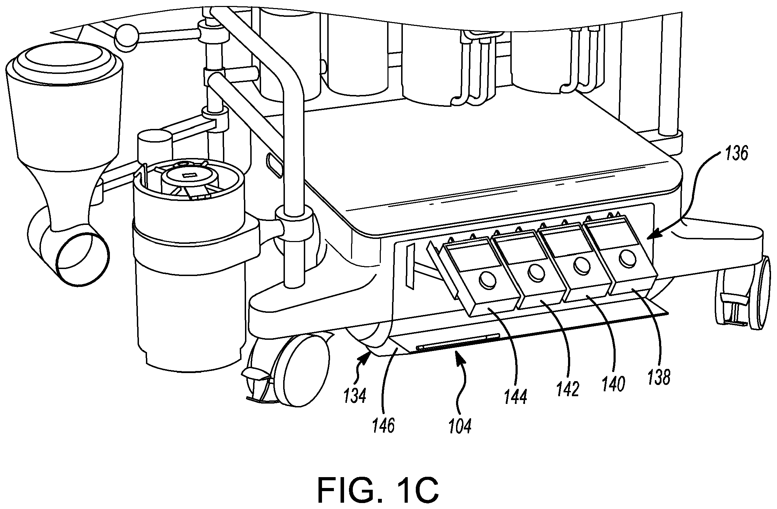

FIG. 1C is a partial perspective view of the base 104 of the trolley 102 depicted in FIGS. 1A and 1B, in accordance with embodiments of the subject matter disclosed herein. As shown in FIG. 1C, the base 104 of the trolley 102 may include a lower housing 134 having an enclosure 136 configured to house one or more ACUs 138, 140, 142, and 144. In embodiments, any number of ACUs may be disposed in the enclosure 136. For example, in embodiments, all of the ACUs for actuators associated with the FILM 100 may be disposed at least partly in the enclosure 136. In embodiments, one or more ACUs may be exposed by being disposed directly on or near the corresponding actuators. According to embodiments, the enclosure 136 may be configured to be closed to protect the ACUs disposed therein, or opened to reveal the ACUs. For example, the lower housing 134 may include a drawer, cabinet, and/or the like. As shown, in embodiments, the lower housing 134 may include a door 146 configured to be opened and closed to selectively expose or conceal the enclosure 136. Each of the ACUs 138, 140, 142, and 144 may be operably connected to a corresponding one of the components 116, 118, 120, 122 (e.g., in cases in which the pump 122 does not include an exposed ACU 126), or 124 (e.g., in cases in which the pump 124 does not include an exposed ACU 128) and/or other actuators.

As is further shown in FIGS. 1A and 1B, the FILM 100 may include any number of other components such as, for example, a venous reservoir 148 (which may be, for example, a component of an extracorporeal circuit that may be used in combination with the FILM 100), an electronic venous occluder (EVO) 150, a peripheral display device 152, a control assembly 154 (which may be interchangeably referred to as "the cockpit"), any number of various types of sensors, and/or the like. According to embodiments, any number of the components discussed herein, others not discussed herein, or aspects of the components (e.g., sensors and/or actuators associated with components) may be operably connected to the peripheral processing unit (not shown), which may be configured to receive parameter data from any one or more of the components, process parameter data, receive control signals from any one or more input devices (e.g., ACU control knobs 130, etc.), provide control signals to any one or more of the components, and/or the like.

In embodiments, the peripheral display device 152 may be operably connected to the peripheral processing unit and configured to present a set of parameter data received from the peripheral processing unit. In embodiments, the peripheral display device 152 may be, include, or be included within a data recording and/or management system. That is, for example, the peripheral display device 152 may include, or be otherwise associated with, a processing unit separate from that of the HLM, and/or may be configured to record and/or display any number of different operative HLM parameters. In some implementations, for example, the peripheral display device 152 may be configured to obtain and record all of the operative HLM parameter values and/or patient parameters provided by any number of additional monitoring devices. The peripheral display device 152 may be configured to present, graphically, representations of any number of the obtained parameter values, changes in parameter values over time, derived parameter values (e.g., values derived from parameter values), and/or the like.

During an operation, the primary focus of a user of the HLM 100 generally is the oxygenator 115 and the venous reservoir 148. Accordingly, embodiments of the subject matter disclosed herein provide a control assembly 154 near those two components 115 and 148 so that the user can access control devices and view displayed parameters without having to move away from, or be distracted from, the oxygenator 115 and venous reservoir 148. According to embodiments, the control assembly 154 may include a control display device 156 and a number of input control devices 158, 160, 162, and 164. In embodiments, the control assembly 154 may include any number of input control devices (e.g., 1 , 2, 3, 4, 5, 6, etc.) and the number of input control devices may be less than or equal to the number of ACUs in the enclosure 136. The control display device 156 may be configured to present a subset of the parameters presented by the peripheral display device 152 and/or the peripheral display device 152 may be configured to present a subset of the subset of parameters presented by the control display device 156. A different subset of the set of parameter data may be displayed by the peripheral display device 152. In embodiments, the peripheral display device 152 may be configured to display real-time waveform traces, while the control display device 156 may be configured to display numerical representations of the same and/or different parameters.

That is, for example, regardless of what is displayed on the peripheral display device 152, the control display device 156 may be configured to display a specified subset of parameter data that is particularly useful and/or important with respect to a procedure being performed. That specified subset of parameter data may be predetermined, based on the type of procedure; dynamically presented, based on a status of the patient and/or device; and/or the like. In embodiments, all of the information configured to be presented on the control display device 156 may be presented simultaneously - that is, without having tabs for accessing screens showing additional information, without requiring menus for accessing screens showing additional information during a procedure, and/or the like. In embodiments, the control display device 156 may include selectable representations presented onscreen that can be used to configure the display such as, for example, by enabling a user to select a display mode corresponding to a particular HLM component (e.g., a centrifugal pump, a roller pump, etc.), to select a particular display module (e.g., a pre-configured set of data fields in a particular arrangement), and/or the like.

According to embodiments, the peripheral display device 152 and/or the control display device 156 may include an input mechanism configured to enable user interaction with one or more features displayed on the display device 152 and/or 156. That is, for example, the peripheral display device 152 and/or the control display device 156 may be, or include, a touchscreen device configured to receive user input. In embodiments, the peripheral display device 152 and/or the control display device 156 may include an input device connected thereto such as, for example, a mouse, a trackpad, a joystick, and/or the like.

According to embodiments, for example, additional data from devices external to the HLM (e.g., blood gas monitors, electrocardiographs, ventilators, patient monitors, etc.) may be displayed on the peripheral display device 152. As indicated above, the peripheral display device 152 may be controlled by a peripheral processing unit that is separate from the central system unit of the HLM. The peripheral processing unit associated with the peripheral display device 152 may be configured to obtain parameter values (e.g., from the central system unit, sensors, actuators, external devices, etc.) and may be configured to collect the data in a database. The peripheral processing unit may be communicatively coupled to the peripheral display device 152, HLM components, and/or external devices. In embodiments, while the peripheral processing unit may be configured to receive data from the central system unit, an interface unit or any other communication port embedded in the HLM, the peripheral processing unit may be configured so as to not send any data to the central system unit, to the interface unit or other communication ports of the HLM. In other embodiments, the peripheral processing unit, the central system unit, the interface unit or any other communication port of the HLM may be configured to exchange data with one another and/or other devices. According to embodiments, a user may select which data is to be stored by which processing or system unit.

The peripheral processing unit associated with the peripheral display device may be configured to allow user interaction therewith, generate reports based on the obtained data, generate printable documents corresponding to a medical procedure, interact with a printer to cause the printer to print such reports, and/or the like. In embodiments, the peripheral processing unit may be configured to generate, and cause the peripheral display device to present, graphs (e.g., trend charts, curves, etc.) and/or other visual representations of any number of various aspects of data received from HLM components and/or external devices. In embodiments the peripheral display device may be configurable such that a user can select certain types of data and/or representations thereof to display, the manner in which it is displayed, and/or the like. In contrast, for example, the control display device 156 may include only limited configurability, if at all. In this manner, the control display device 156 can be relied upon to present representations of data relevant to the HLM's current use. According to other embodiments, the control display device 156 may have any amount of configurability.

In embodiments, each of the input control devices 158, 160, 162, and 164 may be operably connected to one of the actuators and may be configured to receive user input for controlling an operation of the actuator. According to embodiments, the input control devices 158, 160, 162, and 164 may be operably connected to the respective ACUs 138, 140, 142, and 144, in which case, the input control devices 158, 160, 162, and 164 act in parallel to the ACUs, but do not have priority over them in controlling the actuators. In embodiments, the input control devices are directly connected to the respective ACUs, and the ACUs are connected to the respective actuators, such that an actuator can be controlled by an input control device only through an ACU or directly by an ACU. Therefore, the ACU has prevalence over the input control device in controlling the actuator.

As is further shown in FIG. 1A, the HLM 100 may include a connection assembly 166 configured to facilitate connecting at least one HLM component to the HLM base 104. The connection assembly 166 may include any number of different HLM component connectors configured to facilitate removably connecting an HLM component to the HLM base 104. Each HLM component connector may include any number of connection elements configured to facilitate operatively connecting an HLM component to other components of the HLM. In embodiments, the connection elements may include fluid connection elements, energy connection elements, and/or data connection elements. In embodiments, the control assembly 154 may be configured to facilitate configuring the connection assembly 166. According to embodiments, configuring the connection assembly 166 may include, for example, providing input to a control unit via the control assembly 154 that assigns a particular type of HLM component to the connection assembly 166 and/or a connector thereof, assigns a particular HLM component to the connection assembly 166 and/or a connector thereof, causes the control assembly to display a representation of the connector and/or HLM component, causes the control assembly to include data received via the connector to be displayed in a user interface, and/or the like.

According to embodiments, any one or more of the components of the illustrative HLM 100 may be implemented on one or more computing devices. A computing device may include any type of computing device suitable for implementing aspects of embodiments of the disclosed subject matter. Examples of computing devices include specialized computing devices or general-purpose computing devices such "control units," "control assemblies," "workstations," "servers," "hand-held devices," "heart lung machines," "controllers," and the like, all of which are contemplated within the scope of FIG. 1 , with reference to various components of the HLM 100.

In embodiments, a computing device includes a bus that, directly and/or indirectly, couples the following devices: a processing unit, a memory, an input/output (I/O) port, an I/O component, and a power supply. Any number of additional components, different components, and/or combinations of components may also be included in the computing device. The I/O component may include a presentation component configured to present information to a user such as, for example, a display device, a speaker, a printing device, and/or the like, and/or an input component such as, for example, a microphone, a joystick, a satellite dish, a scanner, a printer, a wireless device, a keyboard, a pen, a voice input device, a touch input device, a touch-screen device, an interactive display device, a mouse, and/or the like.

The bus represents what may be one or more busses (such as, for example, an address bus, data bus, or combination thereof). Similarly, in embodiments, the computing device may include a number of processing units, a number of memory components, a number of I/O ports, a number of I/O components, and/or a number of power supplies. Additionally any number of these components, or combinations thereof, may be distributed and/or duplicated across a number of computing devices.

In embodiments, the memory includes computer-readable media in the form of volatile and/or nonvolatile memory and may be removable, nonremovable, or a combination thereof. Media examples include Random Access Memory (RAM); Read Only Memory (ROM); Electronically Erasable Programmable Read Only Memory (EEPROM); flash memory; optical or holographic media; magnetic cassettes, magnetic tape, magnetic disk storage or other magnetic storage devices; data transmissions; and/or any other medium that can be used to store information and can be accessed by a computing device such as, for example, quantum state memory, and/or the like. In embodiments, the memory stores computer-executable instructions for causing the processor to implement aspects of embodiments of system components discussed herein and/or to perform aspects of embodiments of methods and procedures discussed herein.

The computer-executable instructions may include, for example, computer code, machine-useable instructions, and the like such as, for example, program components capable of being executed by one or more processors associated with the computing device. Program components may be programmed using any number of different programming environments, including various languages, development kits, frameworks, and/or the like. Some or all of the functionality contemplated herein may also, or alternatively, be implemented in hardware and/or firmware.

The illustrative HLM 100 shown in FIGS. 1A - 1C is not intended to suggest any limitation as to the scope of use or functionality of embodiments of the present disclosure. The illustrative HLM 100 also should not be interpreted as having any dependency or requirement related to any single component or combination of components illustrated therein. Additionally, various components depicted in FIGS. 1A - 1C may be, in embodiments, integrated with various ones of the other components depicted therein (and/or components not illustrated), all of which are considered to be within the ambit of the present disclosure.

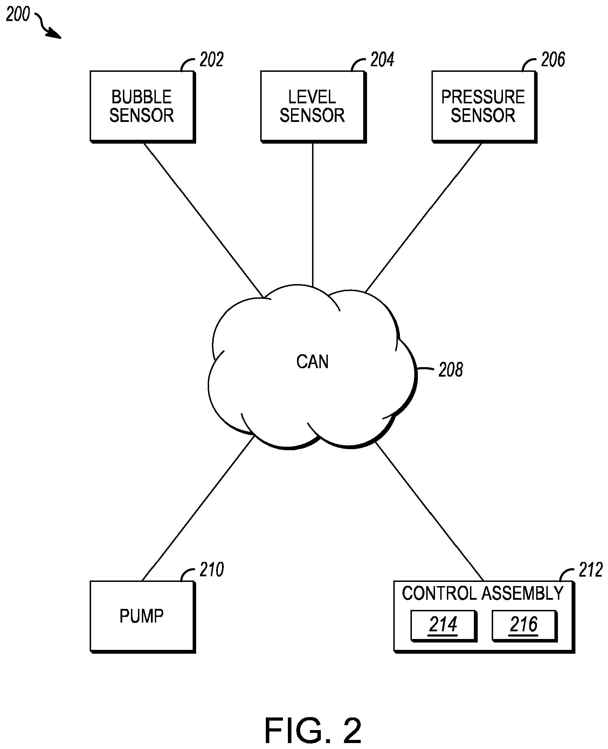

FIG. 2 is a block diagram depicting an illustrative operating environment 200, in accordance with embodiments of the subject matter disclosed herein. According to embodiments, the illustrative operating environment 200 may be implemented on an HLM or aspects thereof such as, for example, the HLM 100 depicted in FIGS. 1A - 1C. As shown, the operating environment 200 includes a number of sensors: a bubble sensor 202, a level sensor 204, and a pressure sensor 206. Each of the sensors 202, 204, and 206 is communicatively coupled to a control area network (CAN) 208. Any number of other types of sensors may be included such as, for example, flow sensors, temperature sensors, blood gas sensors, and/or the like.

A system pump 210 is communicatively coupled to the CAN 208. The system pump 210 may be any kind of fluid pump configured to facilitate performing a perfusion task such as, for example, a roller pump or a centrifugal pump. A control assembly 212 is communicatively coupled to the CAN 208. In embodiments, the control assembly 212 may be, include, be included in, or be similar to the control assembly 154 depicted in FIGS. 1A and 1B. In embodiments, the control assembly 212 may include a processing unit 214 and a memory 216. The processing unit 214 may, for example, be configured to control a control display device by, for example, providing the parameters to display, providing the defining of a layout of display elements, process user input, and/or the like.

In embodiments, the memory includes computer-readable media in the form of volatile and/or nonvolatile memory and may be removable, nonremovable, or a combination thereof. Media examples include Random Access Memory (RAM); Read Only Memory (ROM); Electronically Erasable Programmable Read Only Memory (EEPROM); flash memory; optical or holographic media; magnetic cassettes, magnetic tape, magnetic disk storage or other magnetic storage devices; data transmissions; and/or any other medium that can be used to store information and can be accessed by a computing device such as, for example, quantum state memory, and/or the like. In embodiments, the memory stores computer-executable instructions for causing the processor to implement aspects of embodiments of system components discussed herein and/or to perform aspects of embodiments of methods and procedures discussed herein.

The computer-executable instructions may include, for example, computer code, machine-useable instructions, and the like such as, for example, program components capable of being executed by one or more processors associated with the computing device. Program components may be programmed using any number of different programming environments, including various languages, development kits, frameworks, and/or the like. Some or all of the functionality contemplated herein may also, or alternatively, be implemented in hardware and/or firmware.

The illustrative operating environment 200 shown in FIG. 2 is not intended to suggest any limitation as to the scope of use or functionality of embodiments of the present disclosure. The illustrative operating environment 200 also should not be interpreted as having any dependency or requirement related to any single component or combination of components illustrated therein. Additionally, various components depicted in FIG. 2 may be, in embodiments, integrated with various ones of the other components depicted therein (and/or components not illustrated), all of which are considered to be within the ambit of the present disclosure.

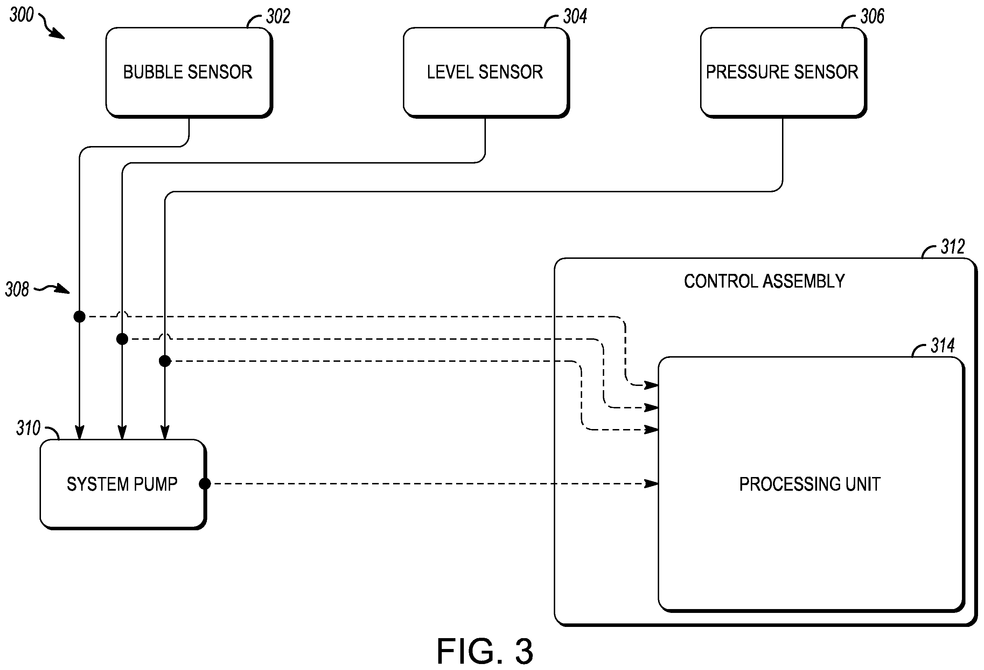

According to embodiments, the instructions may be configured to cause the processing unit 214, upon being executed by the processing unit 214, to facilitate presenting a safety check user interface (UI) on a display device. FIG. 3 is a schematic block diagram depicting an illustrative safety check process, in accordance with embodiments of the subject matter disclosed herein. The process illustrated in FIG. 3 may be implemented, for example, using an operating environment 300, which may be, be similar to, include, or be included in the operating environment 200 depicted in FIG. 2.

As shown, the operating environment 300 includes a bubble sensor 302, a level sensor 304, and a pressure sensor 306, each of which is communicatively coupled to a control area network (CAN) 308. A system pump 310 is also communicatively coupled to the CAN 308. A control assembly 312 (or "cockpit") is communicatively coupled to the CAN 308. The control assembly 312 may include, for example, a control display device and a processing unit 314. In embodiments, the processing unit 314 is configured to provide an FILM system safety check user interface (UI) on a display device. The FILM system safety check UI includes a representation of each of the sensors and may be provided in response to receiving a user selection of a machine profile.

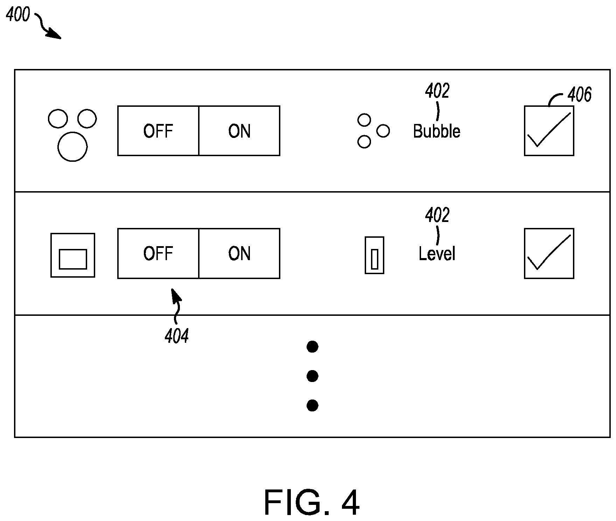

For example, turning briefly to FIG. 4, an illustrative screenshot of an example safety check UI 400 is presented in accordance with embodiments of the subject matter disclosed herein. As shown, the UI 400 may include representations 402 of sensors, as well as a switch 404 depicted next to each representative 402. By interacting with the switch 404, a user may cause the processing unit to activate and deactivate the corresponding sensor. Turning back to FIG. 3, then, the processing unit 314 is configured to receive a user input comprising an indication of a user interaction with a first switch displayed on the UI; and activate the first sensor in response to receiving the indication. Upon activation, the processing unit may be configured to present, via the UI, an indication of the activation of the first sensor.

Additionally, the processing unit 314 may be configured to determine an alarm state of a sensor and present a representation of the alarm state on the control assembly. At the same time, what is shown, for example, in FIG. 4, the UI may include representations 406 of the safety check result concerning that first sensor, in the form of a checkbox that gets checked when the corresponding sensor passes its safety check. According to embodiments, the processing unit may be configured to determine an alarm state of a sensor by receiving a reaction message from the system pump, the reaction message indicating that the system pump acknowledged an alarm state of the first sensor; accessing a first alarm message, via the CAN, wherein the first alarm message is generated by the first sensor and indicates the alarm state of the first sensor; and determining the alarm state of the first sensor based on the first alarm message.

That is, for example, an alarm state can be present if a pump is active and, also, if the pump is not active. For instance, if, with a stopped pump, the bubble sensor detects air along the line, or the level sensor detects air in the reservoir, the alarm condition is present. In such a case, the pump receives the alarm signal and would perform the intended system reaction (e.g. which would stop the pump) if it were running. Then, the pump sends the CAN Message to the control assembly, which would tell the control assembly that the alarm system reaction is being performed by the pump. The control assembly may then check by reading along the CAN messages that the confirmation has really been caused by an induced bubble, level or pressure alarm which would indicate the safety check has been passed and would automatically present it by means of the indication 406. In the illustrated examples, it may automatically check the respective check box in the safety checks table on the UI screen, putting the check box into the "checked" status. The user can see the filled out check box on the screen and be informed that the related check has been completed.

According to embodiments, the signal chain starting from the alarm condition detection by the respective sensor up to the automatic filling out of the check box in the safety checks table is deterministic and can be verified by design verification.

The safety check of a sensor is initiated by switching on the sensor switch 404 in FIG. 4. A sensor alarm may be already present when the safety check starts, or may be artificially induced by the user before or after the safety check start. Additionally, If the checks have not been performed on have not been performed successfully, the assembly requests a confirmation by the user that he/she wants to start the case without having completed the requested checks (e.g. to allow the use in an emergency case with the need to be on bypass as fast as possible). Both the successful check completion and the confirmation of start without checks may also be stored internally in a log file.

The illustrative operating environment 300 shown in FIG. 3 is not intended to suggest any limitation as to the scope of use or functionality of embodiments of the present disclosure. The illustrative operating environment 300 also should not be interpreted as having any dependency or requirement related to any single component or combination of components illustrated therein. Additionally, various components depicted in FIG. 3 may be, in embodiments, integrated with various ones of the other components depicted therein (and/or components not illustrated), all of which are considered to be within the ambit of the present disclosure.

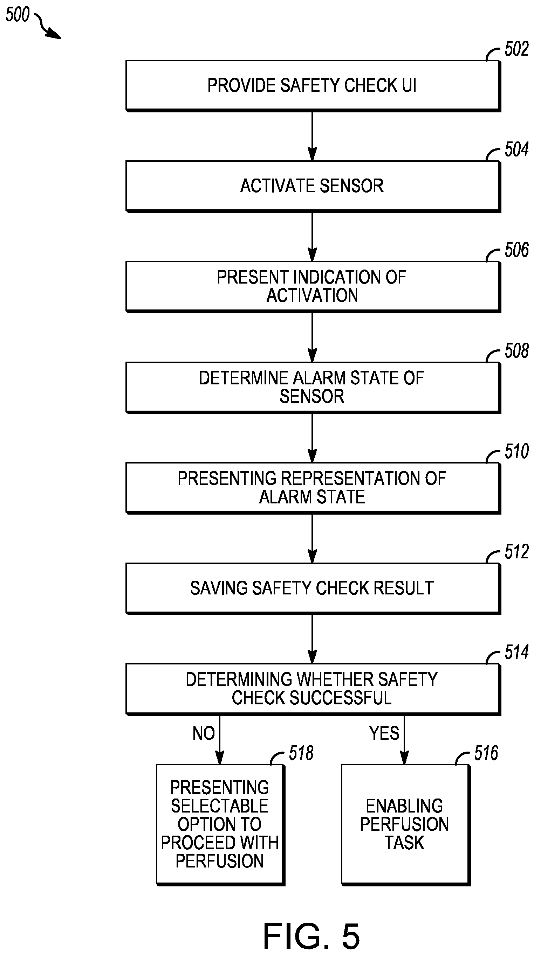

FIG. 5 is a flow diagram depicting an illustrative method 500 of operating a heart lung machine (FILM). According to embodiments, the FILM includes a control area network (CAN); a pump communicatively coupled to the CAN; a number of sensors, wherein each of the plurality of sensors is communicatively coupled to the CAN; and a control assembly communicatively coupled to the CAN. The control assembly includes a control display device and a processing unit. According to embodiments, the FILM may be, be similar to, include, or be included in the FILM 100 depicted in FIGS. 1A-1C and/or the illustrative operating environment 200 depicted in FIG. 2.

As shown in FIG. 5, embodiments of the method 500 include providing an FILM system safety check user interface (UI) on a display device (block 502). The FILM system safety check UI includes a representation of each of the sensors. For example, the UI may include an icon or other graphic representing each sensor, with an interactive switch adjacent the graphic such that a user can activate a sensor by interacting with the switch. In this case, embodiments of the method 500 may further include receiving a user input characterized by an indication of a user interaction with a first switch displayed on the UI and activating the first sensor in response to receiving the indication of the user interaction with the first switch and the start of the safety check for the first sensor (block 504).

As shown in FIG. 5, the method 500 further includes presenting, via the UI, an indication of the activation of the first sensor (block 506), such as, for example, by presenting an interactive switch that is in a switched state. The method 500 further includes determining an alarm state of the first sensor (block 508) and providing a representation of the alarm state of the first sensor on the control assembly (block 510). According to embodiments, determining the alarm state may include, for example, receiving a reaction message from the system pump, the reaction message indicating that the system pump acknowledged an alarm state of the first sensor; accessing a first alarm message, via the CAN, wherein the first alarm message is generated by the first sensor and indicates the alarm state of the first sensor; and determining the alarm state of the first sensor based on the first alarm message. The method further includes saving, in a data management system (DMS) of the HLM, a safety check result corresponding to the first sensor (block 512). According to embodiments, the process described in steps 504-512 may be repeated for any number of different sensors.

According to embodiments, the method 500 further includes determining that the safety check has not been performed successfully (block 514); presenting it, via, the UI, with a selectable option to proceed with a perfusion task (block 518); and receiving a user input that includes a confirmation from the user to proceed with the perfusion task despite the fact that the safety check was not successfully performed. In embodiments, if the safety check is performed successfully, (block 516), the method 500 includes enabling the HLM to perform the perfusion task. According to embodiments, the method 500 may also include saving, in the DMS, (in addition to the safety check result) an indication of the receipt of the confirmation to proceed with the perfusion task.

## Claims

1. A heart lung machine, HLM, (100), comprising:
a control area network, CAN, (208, 308);
a pump (210, 310) communicatively coupled to the CAN;
a plurality of sensors (202, 204, 206, 302, 304, 306), wherein each of the plurality of sensors is communicatively coupled to the CAN; and
a control assembly (212, 312) communicatively coupled to the CAN, the control assembly comprising a control display device and a processing unit (214, 314), the processing unit configured to facilitate a semi-automatic safety check by:
providing an HLM system safety check user interface, UI, (400) on a display device, the HLM system safety check UI comprising a representation of each of the plurality of sensors;
activating a first sensor of the plurality of sensors;
presenting, via the UI, an indication of the activation of the first sensor;
determining an alarm state of the first sensor;
presenting a representation of the alarm state of the first sensor on the control display device;
presenting a safety check result of the first sensor via the Ul;
saving, in a data management system, DMS, or a system log of the HLM, a safety check result corresponding to the first sensor;
activating a second sensor of the plurality of sensors;
presenting, via the UI, an indication of the activation of the second sensor;
determining an alarm state of the second sensor;
presenting a representation of the alarm state of the second sensor on the control display device;
presenting, via the UI, a safety check result of the second sensor; and
saving, in the DMS or the system log of the HLM, a safety check result corresponding to the second sensor;
wherein the processing unit is further configured to facilitate the semi-automatic safety check by:
accessing a first alarm message, via the CAN, wherein the first alarm message is generated by the first sensor and indicates an alarm state of the first sensor; and
determining the alarm state of the first sensor based on the first alarm message;
wherein the pump is configured to:
receive the first alarm message; and
provide, in response to receiving the first alarm message, a reaction message to the processing unit, the reaction message indicating that the pump acknowledged the alarm state of the first sensor; and
perform, in response to receiving the first alarm message, an alarm system reaction, wherein the alarm system reaction comprises ceasing pump operation if the pump is running before receiving the first alarm message;
wherein the processing unit is further configured to:
determine that the alarm system reaction performed by the pump in response to receiving the first alarm message from the first sensor has been executed as intended, thereby identifying a successful safety check result corresponding to the first sensor;
present, via the UI, the successful safety check result; and
enable the HLM to perform a perfusion task.

2. The HLM of claim 1, wherein the processing unit further is configured to:
determine that the alarm system reaction performed by the pump in response to receiving the first alarm message from the first sensor has not been executed as intended, thereby identifying an unsuccessful safety check result corresponding to the first sensor;
present, via the UI, the unsuccessful safety check result;
present, via, the GUI, a selectable option to a proceed with a perfusion task;
receive a user input comprising a confirmation from the user to proceed with the perfusion task despite the fact that the safety check had an unsuccessful result; and
enable the HLM to perform the perfusion task.

3. The HLM of claim 2, wherein the processing unit is further configured to save, in the DMS, or a system log of the HLM, an indication of the receipt of the confirmation to proceed with the perfusion task.

4. The HLM of any of claims 2-3, wherein the processing unit is further configured to save,
in the DMS, or a system log of the HLM, an indication of the enabling of the perfusion task.

5. The HLM of any of claims 1-4, wherein the processing unit is configured to provide the UI
in response to receiving a user selection of a machine profile.

6. The HLM, of claim 1, wherein
the processing unit is further configured to facilitate the semi-automatic safety check by:
accessing a second alarm message, via the CAN, wherein the second alarm message is generated by the second sensor and indicates the alarm state of the second sensor
providing, in response to the second alarm message, a second reaction message from the pump to the processing unit, the second reaction message indicating that the pump acknowledged the alarm state of the second sensor; and
determining, based on the second reaction message from the pump acknowledging the second alarm message from the second sensor, the correct execution, or not, of the intended system reaction, thereby identifying the safety check result corresponding to the second sensor.

7. The HLM of claim 6, wherein the processing unit further is configured to:
determine that the safety check has not been performed successfully if the intended system reaction was not executed as intended;
present, via the GUI, the safety check result with a selectable option to proceed with a perfusion task;
receive a user input comprising a confirmation from the user to proceed with the perfusion task despite the fact that the safety check was not successfully performed;
enable the HLM to perform the perfusion task;
save, in the DMS, or in the system log, an indication of the receipt of the confirmation to proceed with the perfusion task.

8. The HLM of any of claims 1-7, wherein the first sensor comprises a level sensor, a bubble sensor, or a pressure sensor.

9. The HLM of any of claims 1-8, wherein the processing unit is configured to:
receive a user input comprising an indication of a user interaction with a first switch displayed on the UI; and
activate the first sensor in response to receiving the indication of the user interaction with the first switch.

10. A method (500) of operating a heart lung machine, HLM, (100), the HLM comprising a control area network, CAN, (208, 308), a pump (210, 310) communicatively coupled to the CAN, a plurality of sensors (202, 204, 206, 302, 304, 306), wherein each of the plurality of sensors is communicatively coupled to the CAN, and a control assembly (212, 312) communicatively coupled to the CAN, the control assembly comprising a control display device and a processing unit (214, 314), the method comprising:
providing an HLM system safety check user interface, UI, (400) on a display device, the HLM system safety check UI comprising a representation of each of the plurality of sensors;
activating a first sensor of the plurality of sensors;
presenting, via the UI, an indication of the activation of the first sensor;
determining an alarm state of the first sensor;
presenting a representation of the alarm state of the first sensor on the control display device;
presenting, via the UI, a result of the safety check of the first sensor;
saving, in a data management system, DMS, or a system log, of the HLM, the safety check result corresponding to the first sensor;
activating a second sensor of the plurality of sensors;
presenting, via the UI, an indication of the activation of the second sensor;
determining an alarm state of the second sensor;
presenting a representation of the alarm state of the second sensor on the control display device;
presenting, via the UI, a result of the safety check of the second sensor; and
saving, in a data management system, DMS, or a system log, of the HLM, the safety check result corresponding to the second sensor.

11. The method of claim 10, further comprising:
receiving a reaction message from the pump, the reaction message indicating that the pump acknowledged the alarm state of the first sensor;
accessing a first alarm message, via the CAN, wherein the first alarm message is generated by the first sensor and indicates the alarm state of the first sensor; and
determining that an alarm system reaction performed by the pump in response to receiving the first alarm message was executed as intended, thereby identifying and presenting, via the UI, a successful safety check result;
enabling the HLM to perform a perfusion task.

12. The method of either of claims 10 or 11, further comprising:
determining that the pump did not acknowledge the alarm state of the first sensor as intended, thereby identifying and presenting, via the UI, an unsuccessful safety check result;
presenting, via, the GUI, a selectable option to a proceed with a perfusion task;
receiving a user input comprising a confirmation from the user to proceed with the perfusion task despite the fact that the safety check was not successfully performed;
enabling the HLM to perform the perfusion task; and
saving, in the DMS, or a system log, an indication of the receipt of the confirmation to proceed with the perfusion task.

13. The method of any of claims 10-12, wherein the first sensor comprises a level sensor, a bubble sensor, or a pressure sensor.

14. The method of any of claims 10-13, further comprising:
receiving a user input comprising an indication of a user interaction with a first switch displayed on the UI; and
activating the first sensor in response to receiving the indication of the user interaction with the first switch.

15. The method of any of claims 10-14, wherein the HLM is the HLM according to any of claims 1 to 9.

## Patentansprüche

1. Herz-Lungen-Maschine, HLM, (100), mit:
einem Steuerungsbereichsnetzwerk, CAN, (208, 308);
einer Pumpe (210, 310), die kommunizierend mit dem CAN gekoppelt ist;
mehreren Sensoren (202, 204, 206, 302, 304, 306), wobei jeder der mehreren Sensoren kommunizierend mit dem CAN gekoppelt ist; und
einer Steuereinheit (212, 312), die kommunizierend mit dem CAN gekoppelt ist, wobei die Steuereinheit eine Steueranzeigevorrichtung und eine Verarbeitungseinheit (214, 314) aufweist, wobei die Verarbeitungseinheit ausgebildet ist, eine halbautomatische Sicherheitsüberprüfung zu ermöglichen durch:
Bereitstellen einer HLM-System-Sicherheitsprüfungs-Benutzeroberfläche, UI, (400) auf einer Anzeigevorrichtung, wobei die HLM-System-Sicherheitsprüfungs-UI eine Darstellung jedes der mehreren Sensoren aufweist;
Aktivieren eines ersten Sensors der mehreren Sensoren;
Darstellen einer Indikation der Aktivierung des ersten Sensors mittels der Ul;
Bestimmen eines Alarmzustands des ersten Sensors;
Darstellen des Alarmzustands des ersten Sensors auf der Steueranzeigevorrichtung;
Darstellen eines Sicherheitsprüfungsergebnisses des ersten Sensors mittels der Ul;
Speichern eines Sicherheitsprüfungsergebnisses, das dem ersten Sensor entspricht, in einem Datenverwaltungssystem, DMS, oder einem Systemprotokoll der HLM;
Aktivieren eines zweiten Sensors der mehreren Sensoren;
Darstellen einer Anzeige der Aktivierung des zweiten Sensors mittels der Ul;
Bestimmen eines Alarmzustands des zweiten Sensors;
Darstellen einer Darstellung des Alarmzustands des zweiten Sensors auf der Steueranzeigevorrichtung;
Darstellen eines Sicherheitsprüfergebnisses des zweiten Sensors mittels der Ul; und
Speichern eines Sicherheitsprüfergebnisses, das dem zweiten Sensor entspricht, in dem DMS oder in dem Systemprotokoll der HLM;
wobei die Verarbeitungseinheit ferner ausgebildet ist, die halbautomatische Sicherheitsprüfung zu ermöglichen durch
Zugreifen auf eine erste Alarmmeldung über das CAN, wobei die erste Alarmmeldung von dem ersten Sensor erzeugt wird und einen Alarmzustand des ersten Sensors angibt; und
Bestimmen des Alarmzustands des ersten Sensors auf der Grundlage der ersten Alarmmeldung;
wobei die Pumpe ausgebildet ist zum:
Empfangen der ersten Alarmmeldung; und
als Reaktion auf den Empfang der ersten Alarmmeldung, Bereitstellen einer Reaktionsmeldung für die Verarbeitungseinheit, wobei die Reaktionsmeldung angibt, dass die Pumpe den Alarmzustand des ersten Sensors bestätigt hat; und
als Reaktion auf den Empfang der ersten Alarmmeldung, Ausführen einer Alarmsystemreaktion, wobei die Alarmsystemreaktion Beenden des Pumpenbetriebs beinhaltet, wenn die Pumpe vor dem Empfang der ersten Alarmmeldung in Betrieb ist;
wobei die Verarbeitungseinheit ferner ausgebildet ist zum:
Bestimmen, dass die von der Pumpe als Reaktion auf den Empfang der ersten Alarmmeldung aus dem ersten Sensor durchgeführte Alarmsystemreaktion wie vorgesehen ausgeführt wurde, wodurch ein dem ersten Sensor entsprechendes erfolgreiches Sicherheitsprüfergebnis identifiziert wird;
Darstellen des erfolgreiches Sicherheitsprüfergebnis; und
Freigeben der HLM für die Durchführung einer Perfusionsaufgabe.

2. HLM nach Anspruch 1, wobei die Verarbeitungseinheit ferner ausgebildet ist zu bestimmen, dass die von der Pumpe als Reaktion auf den Empfang der ersten Alarmmeldung aus dem ersten Sensor durchgeführte Alarmsystemreaktion nicht wie beabsichtigt ausgeführt wurde, wodurch ein dem ersten Sensor entsprechendes erfolgloses Sicherheitsprüfergebnis identifiziert wird;
Darstellen des erfolglosen Sicherheitsprüfergebnisses mittels der Ul;
Darstellen einer auswählbaren Option zum Fortfahren mit einer Perfusionsaufgabe mittels der Ul;
Empfangen einer Benutzereingabe, die eine Bestätigung des Benutzers enthält, mit der Perfusion forzufahren, obwohl die Sicherheitsprüfung ein erfolgloses Ergebnis hatte; und
Freigeben der HLM für die Durchführung der Perfusionsaugabe.

3. HLM nach Anspruch 2, wobei die Verarbeitungseinheit ferner ausgebildet ist, in dem DMS oder einem Systemprotokoll der HLM eine Angabe des Empfangs der Bestätigung zum Fortfahren mit der Perfusionsaufgabe zu speichern.

4. HLM nach Anspruch 2 oder 3, wobei die Verarbeitungseinheit ferner ausgebildet ist, in dem DMS oder in einem Systemprotokoll der HLM eine Angabe für die Freigabe der Perfusionsaufgabe zu speichern.

5. HLM nach einem der Ansprüche 1-4, wobei die Verarbeitungseinheit ausgebildet ist, die UI als Reaktion auf den Empfang einer Benutzerauswahl eines Maschinenprofils bereitzustellen.

6. HLM nach Anspruch 1, wobei
die Verarbeitungseinheit ferner ausgebildet ist, die halbautomatische Sicherheitsprüfung zu ermöglichen durch:
Zugreifen auf eine zweite Alarmmeldung über das CAN, wobei die zweite Alarmmeldung von dem zweiten Sensor erzeugt wird und den Alarmzustand des zweiten Sensors angibt, Bereitstellen, als Reaktion auf die zweite Alarmmeldung, einer zweiten Reaktionsmeldung von der Pumpe für die Verarbeitungseinheit, wobei die zweite Reaktionsmeldung angibt, dass die Pumpe den Alarmzustand des zweiten Sensors bestätigt hat; und
auf der Grundlage der zweiten Reaktionsmeldung von der Pumpe, die die zweite Alarmmeldung von dem zweiten Sensor bestätigt, Bestimmen der korrekten Ausführung oder Nicht-Ausführung der beabsichtigten Systemreaktion, wodurch das dem zweiten Sensor entsprechende Sicherheitsprüfungsergebnis identifiziert wird.

7. HLM nach Anspruch 6, wobei die Verarbeitungseinheit ferner ausgebildet ist zum:
Bestimmen, dass die Sicherheitsprüfung nicht erfolgreich durchgeführt wurde, wenn die beabsichtigte Systemreaktion nicht wie beabsichtigt ausgeführt wurde;
Darstellen, mittels der UI, des Ergebnisses der Sicherheitsprüfung mit einer auswählbaren Option zum Fortfahren mit einer Perfusionsaufgabe;
Empfangen einer Benutzereingabe, die eine Bestätigung des Benutzers enthält, mit der Perfusionsaufgabe fortzufahren, obwohl die Sicherheitsprüfung nicht erfolgreich durchgeführt wurde;
Freigeben der HLM für die Durchführung der Perfusionsaufgabe;
Speichern, in dem DMS oder in dem Systemprotokoll, einer Angabe für den Empfang der Bestätigung zum Fortfahren mit der Perfusionsaufgabe.

8. HLM nach einem der Ansprüche 1 bis 7, wobei der erste Sensor einen Füllstandssensor, einen Blasensensor oder einen Drucksensor aufweist.

9. HLM nach einem der Ansprüche 1 bis 8, wobei die Verarbeitungseinheit ausgebildet ist zum:
Empfangen einer Benutzereingabe, die eine Angabe bezüglich einer Benutzerinteraktion mit einem ersten Schalter enthält, der auf der UI dargestellt ist; und
Aktivieren des ersten Sensors als Reaktion auf den Empfang der Angabe bezüglich der Benutzerinteraktion mit dem ersten Schalter.

10. Verfahren (500) zum Betreiben einer Herz-Lungen-Maschine, HLM, (100), wobei die HLM ein Steuerungsbereichsnetzwerk, CAN, (208, 308), eine Pumpe (210, 310), die kommunizierend mit dem CAN gekoppelt ist, mehrere Sensoren (202, 204, 206, 302, 304, 306), wobei jeder der mehreren Sensoren kommunizierend mit dem CAN gekoppelt ist, und eine Steuereinheit (212, 312) aufweist, die kommunizierend mit dem CAN gekoppelt ist, wobei die Steuereinheit eine Steueranzeigevorrichtung und eine Verarbeitungseinheit (214, 314) aufweist, wobei das Verfahren umfasst:
Bereitstellen einer HLM-System-Sicherheitsüberprüfungs-Benutzerschnittstelle, UI, (400) auf einer Anzeigevorrichtung, wobei die HLM-System-Sicherheitsüberprüfungs-UI eine Darstellung jedes der mehreren Sensoren aufweist;
Aktivieren eines ersten Sensors der mehreren Sensoren;
Darstellen einer Angabe zur Aktivierung des ersten Sensors mittels der Ul;
Bestimmen eines Alarmzustands des ersten Sensors;
Darstellen einer Darstellung des Alarmzustands des ersten Sensors auf der Steuerungsanzeigevorrichtung;
Darstellen eines Ergebnisses der Sicherheitsüberprüfung des ersten Sensors mittels der Ul;
Speichern des Sicherheitsüberprüfungsergebnisses, das dem ersten Sensor entspricht, in einem Datenverwaltungssystem, DMS, oder einem Systemprotokoll der HLM;
Aktivieren eines zweiten Sensors der mehreren Sensoren;
Darstellen einer Angabe zur Aktivierung des zweiten Sensors mittels der Ul;
Bestimmen eines Alarmzustands des zweiten Sensors;
Darstellen einer Darstellung des Alarmzustands des zweiten Sensors auf der Steuerungsanzeigevorrichtung;
Darstellen eines Ergebnisses der Sicherheitsprüfung des zweiten Sensors mittels der Ul; und
Speichern des Sicherheitsprüfergebnisses für den zweiten Sensor in einem Datenverwaltungssystem, DMS, oder einem Systemprotokoll der HLM.

11. Verfahren nach Anspruch 10, das ferner umfasst:
Empfangen einer Reaktionsmeldung von der Pumpe, wobei die Reaktionsmeldung angibt, dass die Pumpe den Alarmzustand des ersten Sensors bestätigt hat;
Zugreifen auf eine erste Alarmmeldung über das CAN, wobei die erste Alarmmeldung von dem ersten Sensor erzeugt wird und den Alarmzustand des ersten Sensors angibt; und
Bestimmen, dass eine von der Pumpe als Reaktion auf den Empfang der ersten Alarmmeldung durchgeführte Alarmsystemreaktion wie beabsichtigt ausgeführt wurde, wodurch mittels der UI ein erfolgreiches Sicherheitsprüfungsergebnis identifiziert und dargestellt wird;
Freigeben der HLM für die Durchführung einer Perfusionsaufgabe.

12. Verfahren nach Anspruch 10 oder 11, das ferner umfasst:
Bestimmen, dass die Pumpe den Alarmzustand des ersten Sensors nicht wie vorgesehen bestätigt hat, wodurch mittels der UI ein erfolgloses Sicherheitsprüfungsergebnis identifiziert und angezeigt wird;
Darstellen einer auswählbaren Option zum Fortfahren mit einer Perfusionsaufgabe mittels der grafischen Ul;
Empfangen einer Benutzereingabe, die eine Bestätigung des Benutzers enthält, mit der Perfusionsaufgabe fortzufahren, obwohl die Sicherheitsprüfung nicht erfolgreich durchgeführt wurde;
Freigeben der HLM für die Durchführung der Perfusionsaufgabe; und
Speichern einer Angabe des Empfangs der Bestätigung für das Fortfahren mit der Perfusionsaufgabe in dem DMS oder in einem Systemprotokoll.

13. Verfahren nach einem der Ansprüche 10 bis 12, wobei der erste Sensor einen Füllstandssensor, einen Blasensensor oder einen Drucksensor aufweist.

14. Verfahren nach einem der Ansprüche 10 bis 13, das ferner umfasst:
Empfangen einer Benutzereingabe, die eine Angabe einer Benutzerinteraktion mit einem ersten Schalter enthält, der auf der UI dargestellt wird; und
Aktivieren des ersten Sensors als Reaktion auf den Empfang der Anzeige der Benutzerinteraktion mit dem ersten Schalter.

15. Verfahren nach einem der Ansprüche 10 bis 14, wobei die HLM die HLM gemäß einem der Ansprüche 1 bis 9 ist.

## Revendications

1. Machine cœur poumon (MCP) (100), comprenant:
un réseau de zone de commande (CAN) (208, 308) ;
une pompe (210, 310) accouplée de manière à pouvoir communiquer au CAN ;
une pluralité de capteurs (202, 204, 206, 302, 304, 306), dans laquelle chacun de la pluralité de capteurs est accouplé de manière à pouvoir communiquer au CAN ; et
un ensemble de commande (212, 312) accouplé de manière à pouvoir communiquer au CAN, l'ensemble de commande comprenant un dispositif d'affichage de commande et une unité de traitement (214, 314), l'unité de traitement configurée pour faciliter un contrôle de sécurité semi-automatique par les étapes consistant à :
fournir une interface utilisateur de contrôle de sécurité du système MCP (IU) (400) sur un dispositif d'affichage, l'IU du contrôle de sécurité du système MCP comprenant une représentation de chacun de la pluralité de capteurs ;
activer un premier capteur de la pluralité de capteurs ;
présenter, par l'intermédiaire de l'IU, une indication de l'activation du premier capteur ;
déterminer un état d'alarme du premier capteur ;
présenter une représentation de l'état d'alarme du premier capteur sur le dispositif d'affichage de commande ;
présenter un résultat de contrôle de sécurité du premier capteur par l'intermédiaire de l'IU ;
sauvegarder, dans un système de gestion de données, DMS, ou un journal système de la MCP, un résultat de contrôle de sécurité correspondant au premier capteur ;
activer un second capteur de la pluralité de capteurs ;
présenter, par l'intermédiaire de l'IU, une indication de l'activation du second capteur ;
déterminer un état d'alarme du second capteur ;
présenter une représentation de l'état d'alarme du second capteur sur le dispositif d'affichage de commande ;
présenter, par l'intermédiaire de l'IU, un résultat de contrôle de sécurité du second capteur ; et
sauvegarder, dans le DMS ou le journal système de la MCP, un résultat de contrôle de sécurité correspondant au second capteur ;
dans laquelle l'unité de traitement est en outre configurée pour faciliter le contrôle de sécurité semi-automatique par les étapes consistant à :
accéder à un premier message d'alarme, par l'intermédiaire du CAN, dans laquelle le premier message d'alarme est généré par le premier capteur et indique un état d'alarme du premier capteur ; et
déterminer l'état d'alarme du premier capteur sur la base du premier message d'alarme ;
dans laquelle la pompe est configurée pour:
recevoir le premier message d'alarme ; et à
fournir, en réponse à la réception du premier message d'alarme, un message de réaction à l'unité de traitement, le message de réaction indiquant que la pompe a accusé réception de l'état d'alarme du premier capteur ; et
à exécuter, en réponse à une réception du premier message d'alarme, une réaction du système d'alarme, dans laquelle la réaction du système d'alarme comprend l'arrêt du fonctionnement de la pompe si la pompe fonctionne avant de recevoir le premier message d'alarme ;
dans laquelle l'unité de traitement est en outre configurée pour :
déterminer que la réaction du système d'alarme exécutée par la pompe en réponse à la réception du premier message d'alarme du premier capteur a été exécutée tel que prévu, identifiant ainsi un résultat de contrôle de sécurité réussi correspondant au premier capteur ;
présenter, par l'intermédiaire de l'IU, le résultat de contrôle de sécurité réussi ; et
permettre à la MCP d'exécuter une tâche de perfusion.

2. MCP selon la revendication 1, dans laquelle l'unité de traitement est en outre configurée pour :
déterminer que la réaction du système d'alarme exécutée par la pompe en réponse à une réception du premier message d'alarme du premier capteur n'a pas été exécutée tel que prévu, identifiant ainsi un résultat de contrôle de sécurité non réussi correspondant au premier capteur ;
présenter, par l'intermédiaire de l'IU, le résultat du contrôle de sécurité non réussi ;
présenter, par l'intermédiaire de, la GUI, une option pouvant être sélectionnée vers une poursuite avec une tâche de perfusion ;
recevoir une entrée utilisateur comprenant une confirmation de la part de l'utilisateur de poursuivre la tâche de perfusion malgré le fait que le contrôle de sécurité présente un résultat non réussi ; et
permettre à la MCP d'exécuter la tâche de perfusion.

3. MCP selon la revendication 2, dans laquelle l'unité de traitement est en outre configurée pour sauvegarder, dans le DMS, ou un journal système de la MCP, une indication de la réception de la confirmation de poursuivre avec la tâche de perfusion.

4. MCP selon l'une quelconque des revendications 2 à 3, dans laquelle l'unité de traitement est en outre configurée pour sauvegarder, dans le DMS, ou un journal système de la MCP, une indication de l'autorisation de la tâche de perfusion.

5. MCP selon l'une quelconque des revendications 1 à 4, dans laquelle l'unité de traitement est configurée pour fournir l'IU en réponse à une réception d'une sélection utilisateur d'un profil machine.

6. MCP, selon la revendication 1, dans laquelle
l'unité de traitement est en outre configurée pour faciliter le contrôle de sécurité semi-automatique :
en accédant à un second message d'alarme, par l'intermédiaire du CAN, dans laquelle le second message d'alarme est généré par le second capteur et indique l'état d'alarme du second capteur
en fournissant, en réponse au second message d'alarme, un second message de réaction de la pompe vers l'unité de traitement, le second message de réaction indiquant que la pompe a accusé réception de l'état d'alarme du second capteur ; et
en déterminant, sur la base du second message de réaction de la part de la pompe accusant réception du second message d'alarme de la part du second capteur, l'exécution correcte, ou pas, de la réaction du système prévue, identifiant ainsi le résultat de contrôle de sécurité correspondant au second capteur.

7. MCP selon la revendication 6, dans laquelle l'unité de traitement est en outre configurée pour :
déterminer que le contrôle de sécurité n'a pas été exécuté avec succès si la réaction du système prévue n'a pas été exécutée tel que prévu ;
présenter, par l'intermédiaire de la GUI, le résultat du contrôle de sécurité par l'intermédiaire d'une option pouvant être sélectionnée de poursuivre avec une tâche de perfusion ;
recevoir une entrée utilisateur comprenant une confirmation de la part de l'utilisateur de poursuivre avec la tâche de perfusion malgré le fait que le contrôle de sécurité n'a pas été exécuté avec succès ;
permettre à la MCP d'exécuter la tâche de perfusion ;
sauvegarder, dans le DMS, ou dans le journal système, une indication de la réception de la confirmation de poursuivre avec la tâche de perfusion.

8. MCP selon l'une quelconque des revendications 1 à 7, dans laquelle le premier capteur comprend un capteur de niveau, un capteur à bulle, ou un capteur de pression.

9. MCP selon l'une quelconque des revendications 1 à 8, dans laquelle l'unité de traitement est configurée pour :
recevoir une entrée utilisateur comprenant une indication d'une interaction utilisateur avec un premier commutateur affiché sur l'IU ; et
activer le premier capteur en réponse à une réception de l'indication de l'interaction utilisateur avec le premier commutateur.

10. Procédé (500) de fonctionnement d'une machine cœur poumon (MCP) (100), la MCP comprenant un réseau de zone de commande (CAN) (208, 308), une pompe (210, 310) accouplée de manière à pouvoir communiquer au CAN, une pluralité de capteurs (202, 204, 206, 302, 304, 306), dans lequel chacun de la pluralité de capteurs est accouplé de manière à pouvoir communiquer au CAN, et un ensemble de commande (212, 312) accouplé de manière à pouvoir communiquer au CAN, l'ensemble de commande comprenant un dispositif d'affichage de commande et une unité de traitement (214, 314), le procédé comprenant les étapes consistant à :
fournir une interface utilisateur de contrôle de sécurité du système MCP (IU) (400) sur un dispositif d'affichage, l'IU du contrôle de sécurité du système MCP comprenant une représentation de chacun de la pluralité de capteurs ;
activer un premier capteur de la pluralité de capteurs ;
présenter, par l'intermédiaire de l'IU, une indication de l'activation du premier capteur ;
déterminer un état d'alarme du premier capteur ;
présenter une représentation de l'état d'alarme du premier capteur sur le dispositif d'affichage de commande ;
présenter, par l'intermédiaire de l'IU, un résultat du contrôle de sécurité du premier capteur ;
sauvegarder, dans un système de gestion de données, DMS, ou un journal système, de la MCP, le résultat de contrôle de sécurité correspondant au premier capteur ;
activer un second capteur de la pluralité de capteurs ;
présenter, par l'intermédiaire de l'IU, une indication de l'activation du second capteur ;
déterminer un état d'alarme du second capteur ;
présenter une représentation de l'état d'alarme du second capteur sur le dispositif d'affichage de commande ;
présenter, par l'intermédiaire de l'IU, un résultat du contrôle de sécurité du second capteur ; et
sauvegarder, dans un système de gestion de données, DMS, ou un journal système, de la MCP, le résultat de contrôle de sécurité correspondant au second capteur.

11. Procédé selon la revendication 10, comprenant en outre les étapes consistant à :
recevoir un message de réaction de la pompe, le message de réaction indiquant que la pompe a accusé réception de l'état d'alarme du premier capteur ;
accéder à un premier message d'alarme, par l'intermédiaire du CAN, dans lequel le premier message d'alarme est généré par le premier capteur et indique l'état d'alarme du premier capteur ; et
déterminer qu'une réaction du système d'alarme exécutée par la pompe en réponse à une réception que le premier message d'alarme a été exécuté tel que prévu, identifiant et présentant ainsi, par l'intermédiaire de l'IU, un résultat de contrôle de sécurité réussi ;
permettre à la MCP d'exécuter une tâche de perfusion.

12. Procédé selon l'une ou l'autre des revendications 10 ou 11, comprenant en outre les étapes consistant à :
déterminer que la pompe n'a pas accusé réception de l'état d'alarme du premier capteur tel que prévu, identifiant et présentant ainsi, par l'intermédiaire de l'IU, un résultat de contrôle de sécurité non réussi ;
présenter, par l'intermédiaire de, la GUI, une option pouvant être sélectionnée vers une poursuite avec une tâche de perfusion ;
recevoir une entrée utilisateur comprenant une confirmation de la part de l'utilisateur de poursuivre avec la tâche de perfusion malgré le fait que le contrôle de sécurité n'a pas été exécuté avec succès ;
permettre à la MCP d'exécuter la tâche de perfusion ; et
sauvegarder, dans le DMS, ou un journal système, une indication de la réception de la confirmation de poursuivre avec la tâche de perfusion.

13. Procédé selon l'une quelconque des revendications 10 à 12, dans lequel le premier capteur comprend un capteur de niveau, un capteur à bulle, ou un capteur de pression.

14. Procédé selon l'une quelconque des revendications 10 à 13, comprenant en outre les étapes consistant à :
recevoir une entrée utilisateur comprenant une indication d'une interaction utilisateur avec un premier commutateur affiché sur l'IU ; et
activer le premier capteur en réponse à une réception de l'indication de l'interaction utilisateur avec le premier commutateur.

15. Procédé selon l'une quelconque des revendications 10 à 14, dans lequel la MCP est la MCP selon l'une quelconque des revendications 1 à 9.
